# EUROPÄISCHE PATENTANMELDUNG

(11) **EP 3 143 970 A1**
(43) Veröffentlichungstag der Anmeldung: **22.03.2017**
(21) Anmeldenummer: 16177058.1
(22) Anmeldetag: 30.06.2016
(51) Int. Cl.: A61F 5/01

(54) **GELENKVERBINDUNG MIT EINER ANTRIEBSEINRICHTUNG SOWIE VERFAHREN ZUR ANSTEUERUNG EINER SCHWENKBEWEGUNG EINER GELENKVERBINDUNG**

(30) Priorität: 06.07.2015 DE 102015110852
(71) Anmelder: Fraunhofer-Gesellschaft zur Förderung der angewandten Forschung e.V., 80686 München (DE)
(72) Erfinder: Minzenmay, David, 70569 Stuttgart (DE)
(74) Vertreter: Mammel und Maser

(57) **Zusammenfassung**

Die Erfindung betrifft eine Gelenkverbindung (10) mit einer Antriebseinrichtung (15), insbesondere für die Bewegungsunterstützung von biomechanischen Gelenken, wobei die Antriebseinrichtung (15) einen Motor (14) aufweist, zu dessen Gehäuse (13) ein antriebsseitiges Anschlussmittel (11) feststehend angeordnet ist und ein abtriebsseitiges Anschlussmittel (12) an dessen Abtriebselement (17) oder zum Gehäuse (13) drehbar angeordnet ist und eine
Schwenkbegrenzungseinrichtung (19) zwischen dem Abtriebselement (17) und dem abtriebsseitigen Anschlussmittel (12) wirkt und einen freien Schwenkbereich des abtriebsseitigen Anschlussmittels (12) zum Abtriebselement (17) begrenzt, wobei das Abtriebselement (17) mittels der Schwenkbegrenzungseinrichtung (19) das abtriebsseitige Anschlussmittel (12) drehbar innerhalb eines angetriebenen Schwenkbereiches (11) antreibt und der freie Schwenkbereich und der angetriebene Schwenkbereich zumindest teilweise überlagert sind, sowie ein Verfahren zur Ansteuerung einer Schwenkbewegung mit einer solchen Gelenkverbindung (10).

## Beschreibung

Die Erfindung betrifft eine Gelenkverbindung mit einer Antriebseinrichtung, insbesondere für die Bewegungsunterstützung von biomechanischen Gelenken für eine Orthese, sowie ein Verfahren zur Ansteuerung einer Schwenkbewegung einer Gelenkverbindung mit einer Antriebseinrichtung.

Aus der VeröfFentlichung "Ebrahimi, A. et al.: Bionic Upper Orthotics with Integrated EMG Sensory. In: Conference Proceedings of 23rd IEEE International Symposium on Robot and Human Interactive Communication: Human-Robot Co-Existence: Adaptive Interfaces and Systems for Daily Life, Therapy, Assistance and Socially Engaging Interactions, 2014, ISBN 978-1-4799-6763-6, Seiten 716 - 719" ist eine Gelenkverbindung mit einer Antriebsvorrichtung für die Bewegungsunterstützung von biomechanischen Gelenken für eine Orthese bekannt, welche sowohl antriebsseitig als auch abtriebsseitig einen Anschluss aufweist, wobei das antriebsseitige und abtriebsseitige Anschlussmittel schwenkbar zueinander gelagert sind. Zur Ansteuerung der Schwenkbewegung ist zwischen dem antriebsseitigen und dem abtriebsseitigen Anschlussmittel ein Motor vorgesehen. Dadurch kann eine Bewegung des Unterarmes relativ zum Oberarm, beispielsweise zum Heben und Senken eines Gegenstandes, durchgeführt werden.

Aus der DE 102 07 702 C2 ist des Weiteren eine einstellbare Gelenkorthese bekannt, welche ebenfalls ein anschlussseitiges und abschlussseitiges Antriebsmittel umfasst. Diese umfasst auch eine Stelleinrichtung, welche eine in Richtung der Schwenkachse der Gelenkorthese verschiebbare, relativ zum antriebsseitigen Anschluss drehfest angeordnete Verriegelungsscheibe mit einer Innenverzahnung aufweist. Diese Verriegelungsscheibe ist durch axiales Verschieben entlang der Schwenkachse aus einer formschlüssig verriegelten, sich in Eingriff befindenden Position in eine Freigabestellung bewegbar.

Aus der DE 10 2008 024 747 A1 ist eine orthopädische Vorrichtung mit einer Gelenkverbindung bekannt, welche aus einem Oberteil und einem schwenkbar daran angebrachten Unterteil gebildet ist. Diese Gelenkverbindung weist eine Verriegelungseinrichtung auf, die eine Beugebewegung des Oberteils relativ zu dem Unterteil verhindert, wobei die Verriegelungseinrichtung entweder manuell von einem Benutzer verund entriegelbar ist oder diese von einer Sensoreinheit automatisch entriegelt oder entsperrt wird. Die orthopädische Einrichtung wird vornehmlich als Orthese beispielsweise für das Knie- oder Ellenbogengelenk eingesetzt, um eine Beugebewegung dieser Gelenke zu unterstützen oder zu verhindern. Die Beugebewegung dieser orthopädischen Vorrichtung erfolgt nur durch den Benutzer mittels Muskelkraft.

Die US 5,817,040 A offenbart eine Knie- oder Ellenbogenorthese, bestehend aus einem oberen und einem unteren Arm, welche durch ein Gelenk schwenkbar miteinander verbunden sind. Diese Gelenkverbindung ermöglicht sowohl eine manuelle Einstellung eines individuellen Schwenkwinkels als auch eine starre Fixierung der Gelenkverbindung, um eine Schwenkbewegung zu verhindern. Bei dieser Orthese erfolgt somit entweder eine Einschränkung eines Schwenkwinkels durch eine Schwenkbegrenzungseinrichtung oder eine starre Fixierung des Knieoder Ellenbogengelenkes.

Der Erfindung liegt die Aufgabe zugrunde, eine Gelenkverbindung mit einer Antriebseinrichtung insbesondere für eine Orthese vorzuschlagen, die sowohl eine durch die Antriebseinrichtung unterstützte als auch eine antriebsfreie Schwenkbewegung ermöglicht. Desweiteren liegt der Erfindung die Aufgabe zugrunde, ein Verfahren zur Ansteuerung einer solchen Schwenkbewegung vorzuschlagen.

Diese Aufgabe wird durch eine Gelenkverbindung mit einer Antriebseinrichtung, insbesondere für die Bewegungsunterstützung von biomechanischen Gelenken für Orthesen, gelöst, welche ein antriebsseitiges Anschlussmittel und ein abtriebsseitiges Anschlussmittel umfasst, die schwenkbar zueinander gelagert sind, wobei die Antriebseinrichtung einen Motor aufweist, zu dessen Gehäuse das antriebsseitige Anschlussmittel feststehend angeordnet ist und das abtriebsseitige Anschlussmittel an dessen Abtriebselement oder zum Gehäuse drehbar angeordnet ist, wobei eine Schwenkbegrenzungseinrichtung zwischen dem Abtriebselement und dem abtriebsseitigen Anschlussmittel wirkt, welche wenigstens ein Anschlagelement und wenigstens ein an dem zumindest einen Anschlagelement zur Anlage kommendes Antriebselement umfasst, und einen freien Schwenkbereich des abtriebsseitigen Anschlussmittels zum Abtriebselement begrenzt und das Abtriebselement mittels der Schwenkbegrenzungseinrichtung das abtriebsseitige Anschlussmittel drehbar innerhalb eines angetriebenen Schwenkbereiches antreibt und der freie Schwenkbereich und der angetriebene Schwenkbereich zumindest teilweise überlagert sind. Die schwenkbare Anordnung des antriebsseitigen Anschlussmittels und des abtriebsseitigen Anschlussmittels ermöglicht eine kompakte Ausgestaltung der Gelenkverbindung, indem die beiden Anschlussmittel unmittelbar nebeneinander liegend oder ineinander greifend anordenbar sind. Durch die Schwenkbegrenzungseinrichtung ist eine freie Schwenkbewegung des abtriebsseitigen Anschlussmittels zum Abtriebselement ermöglicht, ohne dass eine angetriebene Drehbewegung des Abtriebsmittels erforderlich ist. Der Schwenkwinkel des abtriebsseitigen Anschlussmittels wird durch die Schwenkbegrenzungseinrichtung begrenzt. Zusätzlich ist durch den angetriebenen Schwenkbereich eine antriebsunterstützte Schwenkbewegung des abtriebsseitigen Anschlussmittels ermöglicht. Durch die motorische Antriebsunterstützung sind größere Hebelkräfte durch die Gelenkverbindung erreichbar. Diese können beispielsweise dazu dienen, eine Bewegung eines biomechanischen Gelenkes zu unterstützen. Durch die teilweise Überlagerung des freien Schwenkbereiches und des angetriebenen Schwenkbereiches wird erreicht, dass auch während der angetriebenen Schwenkbewegung eine freie Schwenkbewegung ermöglicht ist.

In einer bevorzugten Ausführungsform der Gelenkverbindung ist vorgesehen, dass das abtriebsseitige Anschlussmittel von dem Abtriebselement durch eine Radiallagerung drehbar aufgenommen ist. Die Radiallagerung ermöglicht die Schwenkbewegung des abtriebsseitigen Anschlussmittels zum Abtriebselement, die bevorzugt um eine gemeinsame Achse erfolgt. Durch die Radiallagerung des abtriebsseitigen Anschlussmittels unmittelbar an dem Abtriebselement wird der Einsatz zusätzlicher Verbindungselemente zwischen dem Anschlussmittel und dem Abtriebselement vermieden. Dadurch wird eine kompakte Bauform der Gelenkverbindung erreicht.

In einer weiteren bevorzugten Ausführungsform der Gelenkverbindung ist vorgesehen, dass das abtriebsseitige Anschlussmittel von dem Gehäuse oder dem antriebsseitigen Anschlussmittel durch die Radiallagerung drehbar aufgenommen ist. Durch diese konstruktive Ausgestaltung können noch kompaktere Bauformen der Gelenkverbindung ausgebildet werden.

In einer vorteilhaften Ausgestaltung der Gelenkverbindung ist vorgesehen, dass das abtriebsseitige Anschlussmittel unmittelbar auf einer radialen Lauffläche des Abtriebselementes, des Gehäuses oder dem antriebsseitigen Anschlussmittel abgestützt ist. Durch diese Gleitlagerung zwischen dem abtriebsseitigen Anschlussmittels und der radialen Lauffläche des Abtriebselementes sind hohe Tragkräfte der Lagerung erreichbar. Zudem werden zusätzliche Bauteile zur Lagerung, beispielsweise ein Wälzlager vermieden, sodass auch hierdurch ein kompaktes Bauvolumen der Lagerung erreicht ist. Die Gleitlagerung des abtriebsseitigen Anschlussmittels bietet zusätzlich den Vorteil einer hohen Lebensdauer und ist unempfindlich gegenüber äußeren Einwirkungen, wie z.B. Stöße.

Bevorzugt ist das abtriebsseitige Anschlussmittel durch eine Schulter oder ein Sicherungselement zur Lauffläche des Abtriebselementes, des Gehäuses oder dem antriebsseitigen Anschlussmittel in axialer Richtung gesichert, um ein Lagerspiel des abtriebsseitigen Anschlussmittels zu begrenzen und ein Ablösen des abtriebsseitigen Anschlussmittel von der Lagerstelle zu verhindern.

Eine vorteilhafte Weiterbildung der Gelenkverbindung sieht vor, dass das abtriebsseitige Anschlussmittel und das Abtriebselement um eine gemeinsame Drehachse schwenkbar gelagert sind, welche der gemeinsamen Achse des antriebsseitigen Anschlussmittels und des abtriebsseitigen Anschlussmittels entspricht. Dadurch sind alle drehbar zueinander gelagerten Bauteile auf einer gemeinsamen Drehachse angeordnet, wodurch eine noch kompaktere Bauform der Gelenkverbindung erreicht ist und die Bauteile zumindest teilweise in einem gemeinsamen zylindrischen Gehäuse vorgesehen sein können.

Die Schwenkbegrenzungseinrichtung umfasst gemäß einer bevorzugten Ausführungsform der Gelenkverbindung wenigstens ein, vorzugsweise zwei zueinander beabstandete Anschlagelemente und wenigstens ein an dem zumindest einem Anschlagelement zur Anlage kommendes Antriebselement. Indem das Antriebselement an das wenigstens eine Anschlagelement der Schwenkbegrenzungseinrichtung zur Anlage gebracht wird, ist die freie Schwenkbewegung des abtriebsseitigen Anschlussmittels zumindest in eine Schwenkrichtung begrenzt. Das Vorsehen eines weiteren, zweiten Anschlagelementes begrenzt die freie Schwenkbewegung zusätzlich in eine gegensinnige Schwenkrichtung des abtriebsseitigen Anschlussmittels, sodass durch das Zusammenwirken des Antriebselementes und den beiden Anschlagelementen der freie Schwenkbereich in beide Schwenkrichtungen begrenzt ist. Durch den Abstand zwischen den beiden Anschlagelementen zueinander ist ein Winkel für einen freien Schwenkbereich definiert. Durch eine Veränderung dieses Abstandes kann der freie Schwenkwinkel vergrößert oder verkleinert werden und wird dadurch an einen speziellen Anwendungsbereich angepasst.

Eine vorteilhafte Ausgestaltung der Gelenkverbindung sieht desweiteren vor, dass entweder das Antriebselement an dem Abtriebselement angeordnet ist und die wenigstens zwei Anschlagelemente an dem abtriebsseitigen Anschlussmittel vorgesehen sind oder alternativ das Antriebselement an dem abtriebsseitigen Anschlussmittel angeordnet ist und die wenigstens zwei Anschlagelemente an dem Abtriebselement vorgesehen sind. Dadurch ist mit nur einem zusätzlichen Bauteil, dem Antriebselement, eine unmittelbare Wirkverbindung zwischen dem Abtriebselement und dem abtriebsseitigen Anschlussmittel geschaffen. Diese Wirkverbindung kann durch einen konstruktiv einfachen Aufbau erreicht werden und weist eine geringe Fehleranfälligkeit auf.

Eine weitere vorteilhafte Ausgestaltung der Gelenkverbindung sieht vor, dass die Anschlagelemente durch einen Schlitz oder eine Nut begrenzende Stirnflächen gebildet sind, welche an dem abtriebsseitigen Anschlussmittel vorgesehen ist und in welche das vorzugsweise stiftförmige Antriebselement eingreift. Alternativ können die Anschlagelemente durch an dem abtriebsseitigen Anschlussmittel vorgesehene Zähne, Laschen oder Anschläge gebildet sein. In einer weiteren alternativen Ausführungsform können die Anschlagelemente durch ein abtriebsseitiges kreissegmentförmiges Anschlussmittel gebildet sein. Durch diese konstruktiv unterschiedlichen Ausführungsformen der Schwenkbegrenzungseinrichtung ist die Gelenkverbindung an unterschiedliche Einsatzgebiete anpassbar, da je nach Ausführungsform der Abstand zwischen den Anschlagelementen, den Zähnen, Laschen oder Anschlägen oder dem kreissegmentförmigen Anschlussmittel individuell verändert werden kann. Zudem sind durch diese verschiedenen Ausführungsformen unterschiedliche Drehmomente übertragbar.

In einer bevorzugten Ausführungsform der Gelenkverbindung wird beim Anliegen des Antriebselementes an einem der beiden Anschlagelemente das abtriebsseitige Anschlussmittel in eine der beiden Drehrichtungen des Abtriebselementes innerhalb eines angetriebenen Schwenkbereiches schwenkbar angetrieben. Sobald das Antriebselement an einem der beiden Anschlagelemente anliegt und von der Antriebseinrichtung in eine Drehbewegung versetzt wird, wird eine angetriebene Schwenkbewegung ausgeführt. Während der angetriebenen Schwenkbewegung in eine Drehrichtung ist durch die Schwenkbegrenzungseinrichtung zumindest in dieselbe Drehrichtung gleichzeitig die freie Schwenkbewegung möglich.

Eine weitere Ausgestaltung der Gelenkverbindung sieht vor, dass die Antriebseinrichtung zwischen einem Rotor des Motors und dem damit wirkverbundenen Abtriebselement ein Getriebe aufweist, welches insbesondere als Untersetzungsgetriebe ausgebildet ist. Durch das Getriebe wird die hohe Drehzahl des Motors und somit die hohe Rotationsgeschwindigkeit des Rotors reduziert und ein geringes Drehmoment des schnelllaufenden Motors in ein hohes Drehmoment des Abtriebselementes umgesetzt. Dadurch können große Hebelkräfte der Gelenkverbindung erreicht werden. Als Getriebe wird beispielsweise ein sogenanntes Harmonic Drive Getriebe eingesetzt, welches ein hohes Übersetzungsverhältnis, bei einer gleichzeitig sehr kompakten Bauform aufweist.

Vorteilhafterweise ist der Motor der Gelenkverbindung als schnelllaufender Elektromotor ausgebildet, welcher gegenüber langsam laufenden Motoren den Vorteil eines geringen Bauvolumens aufweist. Der Einsatz eines solchen schnelllaufenden Motors bietet insbesondere bei einem mobilen Einsatz der Gelenkverbindung den Vorteile, dass kompakte Abmessungen des Motors und ein geringes Gesamtgewicht der Gelenkverbindung erzielt werden.

Eine bevorzugte Weiterbildung der Gelenkverbindung sieht vor, dass wenigstens ein Sensorelement zur Detektion der Stellposition des wenigstens einen Antriebselementes und/oder der Winkelstellung des abtriebsseitigen Anschlussmittels und zur Ansteuerung des Motors vorgesehen ist. Durch das Sensorelement ist eine permanente oder temporäre Überwachung der Position des Antriebselementes bzw. des abtriebsseitigen Anschlussmittels ermöglicht. Beispielsweise kann dadurch bei einer erforderlichen Antriebsunterstützung der Motor angesteuert werden oder bei Erreichen einer bestimmten Winkelstellung des abtriebsseitigen Anschlussmittels der Motor stillgesetzt werden.

In einer weiteren bevorzugten Weiterbildung der Gelenkverbindung ist vorgesehen, dass wenigstens ein Sensor, vorzugsweise ein Drehmomentsensor, Dehnungsmessstreifen, Elektromyographie-Sensor (EMG-Sensor) oder eine externe Druckplatine, zur Ansteuerung des Motors vorgesehen ist. Durch diesen Sensor ist es ermöglicht, mit einem externen Signal den Motor anzusteuern. Beispielsweise registriert der Drehmomentsensor oder der Dehnungsmessstreifen bei einer Schwenkbewegung ein Überschreiten eines vorbestimmten Drehmomentes oder einer Hebelkraft, sodass bei Erreichen dieses Drehmomentes oder dieser Hebelkraft der Drehmomentsensor oder der Dehnungsmessstreifen ein Signal ausgibt und der Motor über eine elektronische Regelung angesteuert wird, um die Schwenkbewegung des abtriebsseitigen Anschlussmittels zusätzlich motorunterstützend anzutreiben. Alternativ kann ein Elektromyographie-Sensor ein Signal ausgeben, sodass über einen muskulären oder nervalen Impuls eine Ansteuerung des Motors erfolgt. Alternativ ist der Motor über eine externe Druckplatine ansteuerbar, sodass das Signal über eine manuelle Betätigung der Druckplatine ausgegeben wird.

Der freie Schwenkbereich des abtriebsseitigen Anschlussmittels ist in einer bevorzugten Ausführungsform der Gelenkverbindung gleich groß oder kleiner als ein einstellbarer Schwenkwinkel des abtriebsseitigen Anschlussmittels. Dies bietet den Vorteil, dass durch das abtriebsseitige Anschlussmittel eine freie Schwenkbewegung über den gesamten Schwenkwinkel des abtriebsseitigen Anschlussmittels ermöglicht ist. Daraus resultiert ein größtmöglicher freier Schwenkbereich.

In einer bevorzugten Ausführungsform der Gelenkverbindung ist vorgesehen, dass der einstellbare Schwenkwinkel des abtriebsseitigen Anschlusselementes entweder durch wenigstens ein, vorzugsweise zwei Endanschläge am Gehäuse oder am antriebsseitigen Anschlussmittel oder durch eine elektronische Regelung des Motors begrenzt ist. Durch die Begrenzung des einstellbaren Schwenkwinkels des abtriebsseitigen Anschlusselementes kann die Schwenkbewegung an den spezifischen Anwendungsfall angepasst werden, bei welchem beispielsweise eine Schwenkbewegung nur in einem vorbestimmten Winkelbereich ausgeführt werden soll. Bei der Bewegungsunterstützung eines biomechanischen Gelenkes wird dadurch beispielsweise ein Überstrecken des Gelenkes verhindert. Ebenso können sowohl die Endanschläge als auch die elektronische Regelung des Motors zur Begrenzung des maximalen Schwenkwinkels vorgesehen sein, sodass die elektronische Regelung des Motors einen Stillstand der Schwenkbewegung vor Erreichen eines der Endanschläge bewirkt und die Endanschläge als Sicherheitselemente zur Begrenzung des einstellbaren Schwenkwinkels dienen, beispielsweise bei einer Störung der elektronischen Regelung.

Die der Erfindung zugrunde liegende Aufgabe wird desweiteren durch ein Verfahren zur Ansteuerung einer Schwenkbewegung von einem abtriebsseitigen Anschlussmittel relativ zu einem antriebsseitigen Anschlussmittel einer Gelenkverbindung mit einer Antriebseinrichtung nach einer der vorbeschriebenen Ausführungsformen gelöst, wobei ein Motor der Antriebseinrichtung durch einen Sensor angesteuert wird und der Motor eine Drehbewegung des Antriebselementes ansteuert, sodass dieses zur Anlage an einem Anschlagelement einer Schwenkbegrenzungseinrichtung übergeführt wird, welche zwischen dem Abtriebselement und dem abtriebsseitigen Anschlussmittel wirkt und das abtriebsseitige Anschlussmittel geschwenkt wird oder bei Anlage des Antriebselementes am Anschlagelement das abtriebsseitige Anschlussmittel geschwenkt wird und der Motor zum Antrieb der Schwenkbewegung des abtriebsseitigen Anschlussmittels bei Erreichen einer Endposition innerhalb eines angetriebenen Schwenkbereiches des Antriebselementes oder bei Anlage an einem dem angetriebenen Schwenkbereich zugeordneten Endanschlag stillgesetzt wird. Durch das mit dem Anschlagelement des abtriebsseitigen Anschlussmittels in Anlage gebrachten Antriebselementes wird eine freie Schwenkbewegung durch die Antriebseinrichtung motorisch unterstützt, sodass eine kraftunterstützte Schwenkbewegung, die auch größere Hebelkräfte mit dem abtriebsseitigen Anschlussmittel aufweisen kann, erreichbar sind.

In einer bevorzugten Ausführungsform des Verfahrens wird bei Erreichen der Endposition die angetriebene Schwenkbewegung durch eine elektronische Regelung, durch den Endanschlag oder durch einen Kontakt zu einem separaten Sensor oder Schalter automatisch beendet, indem der Motor abgeschaltet wird.

Eine bevorzugte Weiterbildung des Verfahrens sieht vor, dass als Sensor zur Ansteuerung des Motors ein Drehmomentsensor, Dehnungsmessstreifen, Elektromyographiesensor (EMG-Sensor) oder eine externe Druckplatine eingesetzt und der Sensor durch ein Belastung, einen muskulären oder nervalen Impuls oder eine aktive Betätigung der Druckplatine aktiviert wird. Die Ansteuerung des Motors zur Ausführung der Schwenkbewegung des abtriebsseitigen Anschlusselementes erfolgt somit durch ein externes Signal, welches beispielsweise bei einer Belastung der Gelenkverbindung durch ein Drehmoment oder eine Hebelkraft von dem Drehmomentsensor oder dem Dehnungsmessstreifen initiiert wird oder bei einem muskulären Reiz durch den EMG-Sensor oder durch eine manuelle Betätigung der Druckplatine.

In einer vorteilhaften Weiterbildung des Verfahrens ist vorgesehen, dass nach einer kraftunterstützten Schwenkbewegung des abtriebsseitigen Anschlussmittels innerhalb des angetriebenen Schwenkbereiches, der Motor angesteuert wird, sodass das Antriebselement in eine Ausgangsposition zurückgeführt wird, welche vorzugsweise mittig zwischen zwei Endposition des angetriebenen Schwenkbereiches oder zwischen den Endanschlägen liegt. Dadurch ist sichergestellt, dass nach dem Ausführen einer kraftunterstützten Schwenkbewegung ebenfalls eine antriebsunterstützte Rückschwenkbewegung in die gegensinnige Schwenkrichtung erfolgt.

Das Verfahren sieht vorteilhafterweise vor, dass die Lage des freien Schwenkbereiches des abtriebsseitigen Anschlussmittels durch die Ausgangsposition oder eine davon abweichende Stellposition des Antriebselementes festgelegt ist. Dadurch ist der freie Schwenkbereich innerhalb des möglichen bzw. einstellbaren Schwenkwinkels des abtriebsseitigen Anschlussmittels in Abhängigkeit von der Position des Antriebselementes frei veränderbar wodurch eine hohe Flexibilität der Lage des freien Schwenkbereiches gegeben ist.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine perspektivische Vorderansicht der erfindungsgemäßen Gelenkverbindung,
Figur 2 eine perspektivische Ansicht der Gelenkverbindung gemäß Figur 1 von hinten,
Figur 3 eine Schnittansicht der Gelenkverbindung gemäß Figur 1,
Figur 4 eine schematische Draufsicht der Gelenkverbindung in einer Ausgangsposition,
Figur 5 eine schematische Draufsicht der Gelenkverbindung in einer ersten Endposition eines freien Schwenkbereiches,
Figur 6 eine schematische Draufsicht der Gelenkverbindung in einer zweiten Endposition des freien Schwenkbereiches,
Figur 7 eine schematische Draufsicht auf die Gelenkverbindung in einer ersten Endposition eines angetriebenen Schwenkbereiches,
Figur 8 eine schematische Draufsicht auf die Gelenkverbindung in einer zweiten Endposition des angetriebenen Schwenkbereiches,
Figur 9 eine schematische Draufsicht einer alternativen Ausführungsform einer Schwenkbegrenzungseinrichtung der Gelenkverbindung,
Figur 10 eine schematische Draufsicht auf einer zweiten alternativen Ausführungsform der Schwenkbegrenzungseinrichtung der Gelenkverbindung,
Figur 11 eine schematische Draufsicht auf einer dritten alternativen Ausführungsform der Schwenkbegrenzungseinrichtung der Gelenkverbindung,
Figur 12 eine schematische Draufsicht auf einer vierten alternativen Ausführungsform der Schwenkbegrenzungseinrichtung der Gelenkverbindung,
Figur 13 eine schematische Draufsicht auf einer fünften alternativen Ausführungsform der Schwenkbegrenzungseinrichtung der Gelenkverbindung und
Figur 14 eine schematische Draufsicht einer sechsten alternativen Ausführungsform der Schwenkbegrenzungseinrichtung der Gelenkverbindung.

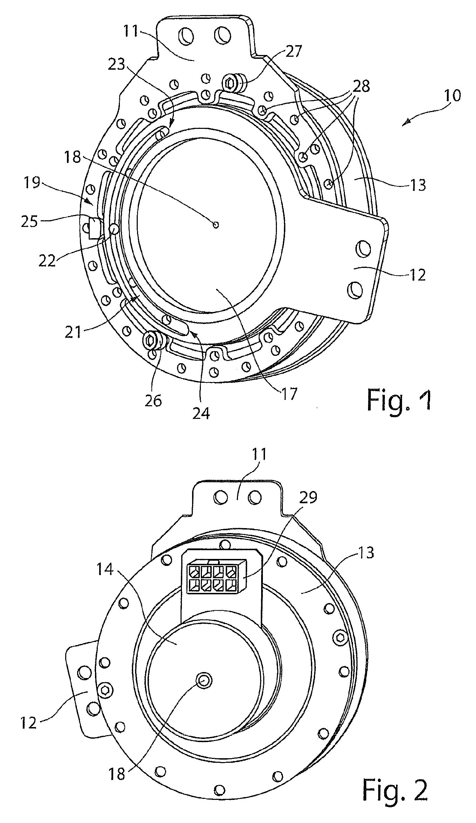

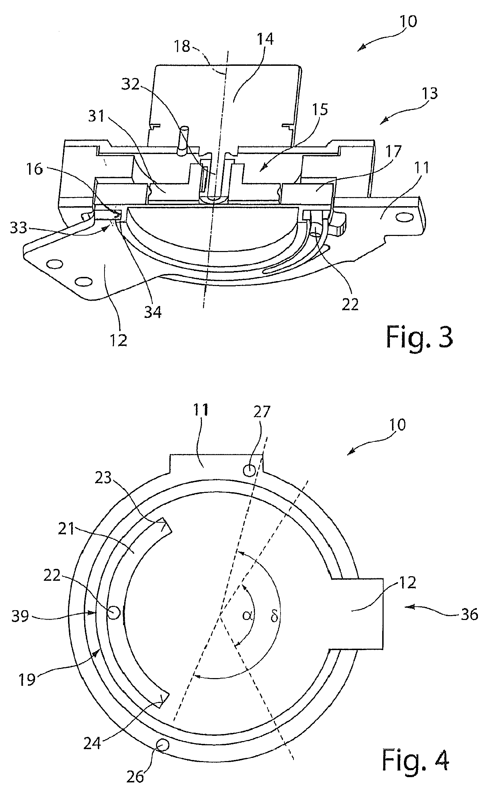

Figur 1 zeigt eine perspektivische Vorderansicht der erfindungsgemäßen Gelenkverbindung 10. Die Gelenkverbindung 10 ist durch ein antriebsseitiges Anschlussmittel 11 und ein abtriebsseitiges Anschlussmittel 12 gebildet, die durch eine Antriebseinrichtung 15 miteinander wirkverbunden sind. Die Antriebseinrichtung 15 umfasst einen Motor 14, welcher an der Rückseite eines Gehäuses 13 der Gelenkverbindung 10 angeordnet und beispielsweise als Elektromotor ausgebildet ist. Die Anordnung des Motors 14 an dem Gehäuse 13 geht aus den Figuren 2 und 3 hervor. Das antriebsseitige Anschlussmittel 11 und das abtriebsseitige Anschlussmittel 12 sind um eine gemeinsame Achse schwenkbar angeordnet.

An dem zylindrischen Gehäuse 13 ist das antriebsseitige Anschlussmittel 11 feststehend angeordnet. In der Darstellung gemäß Figur 1 ist das antriebsseitige Anschlussmittel 11 unmittelbar an dem Gehäuse 13 vorgesehen. Alternativ kann das antriebsseitige Anschlussmittel 11 mittelbar, beispielsweise über ein weiteres Verbindungselement oder durch Anbringen an einem anderen Bauteil der Gelenkverbindung 10, mit dem Gehäuse 13 verbunden sein. Das antriebsseitige Anschlussmittel 11 ist ringscheibenförmig ausgestaltet und weist einen Montageabschnitt zum Anbringen eines nicht näher dargestellten weiteren Bauteils, beispielsweise eines Armes, auf.

Das abtriebsseitige Anschlussmittel 12 ist ebenfalls ringscheibenförmig ausgestaltet und umfasst einen Montageabschnitt zum Anbringen eines weiteren, nicht näher dargestellten Bauteils. Der Außendurchmesser des abtriebsseitigen Anschlussmittels 12 entspricht im Wesentlichen dem Innendurchmesser des antriebsseitigen Anschlussmittels 11, kann jedoch ebenso einen kleineren oder größeren Durchmesser aufweisen.

Mit einer nach innen weisenden Ringfläche des abtriebsseitigen Anschlussmittels 12 ist dieses unmittelbar auf einer radialen Lauffläche 16 eines Abtriebselementes 17 aufgesetzt. Durch die radiale Lauffläche 16 ist eine Radiallagerung 33 für das abtriebsseitige Anschlussmittel 12 gebildet, durch welche dieses von dem Abtriebselement 17 schwenkbar aufgenommen ist. Dadurch sind das abtriebsseitige Anschlussmittel 12 und das Abtriebselement 17 um eine gemeinsame Drehachse 18 schwenkbar gelagert. Diese gemeinsame Drehachse 18 entspricht der gemeinsamen Achse des antriebsseitigen Anschlussmittels 11 und des abtriebsseitigen Anschlussmittels 12.

In einer alternativen Ausführungsform der Gelenkverbindung 10 sind das antriebsseitige Anschlussmittel 11 und das abtriebsseitige Anschlussmittel 12 versetzt zueinander angeordnet, sodass durch den Versatz die Achsen der beiden Anschlussmittel 11, 12 parallel zueinander beabstandet sind.

In einer weiteren Ausführungsform der Gelenkverbindung 10 sind das antriebsseitige Anschlussmittel 11 und das abtriebsseitige Anschlussmittel 12 geneigt zueinander ausgerichtet, wodurch zwischen den beiden Achsen der Anschlussmittel 11, 12 ein Neigungswinkel gebildet ist.

Figur 2 zeigt eine perspektivische Ansicht der Gelenkverbindung 10 von hinten. Aus dieser Ansicht wir deutlich, dass der zylinderförmige Motor 14 an der Rückseite des Gehäuses 13 angeordnet ist. Der Motor 14 ist in der Weise zu dem Gehäuse 13 ausgerichtet, dass dessen Mittelachse auf der gemeinsamen Drehachse 18 des antriebsseitigen und abtriebsseitigen Anschlussmittels 11, 12 angeordnet ist. Der Motor 14 weist eine Schnittstelle 29 auf, über welche dieser mit Energie versorgt und angesteuert wird.

Figur 3 zeigt eine schematisch vereinfachte Schnittansicht der Gelenkverbindung 10. Die Schnittansicht verdeutlicht die Antriebseinrichtung 15, welche durch den Motor 14 mit einem Rotor 32, ein Getriebe 31 sowie das daran angeordnete Abtriebselement 17 und dem davon aufgenommenen Antriebselement 22 gebildet ist. Der Motor 14 ist mit der Mittelachse des Rotors 32 auf der gemeinsamen Drehachse 18 des antriebsseitigen und abtriebsseitigen Anschlussmittels 11, 12 angeordnet. Als Getriebe 31 kann beispielsweise ein sogenanntes Harmonic Drive Getriebe eingesetzt werden, welches besonders hohe Untersetzungsverhältnisse, bei einer sehr kompakten Bauweise ermöglicht.

Aus Figur 3 geht zudem die Radiallagerung 33 des abtriebsseitigen Anschlussmittels 12 an dem Abtriebselement 17 hervor. Die Radiallagerung 33 ist durch die radiale Lauffläche 16 gebildet, auf welcher das abtriebsseitige Anschlussmittel 12 drehbar angeordnet ist. Zur axialen Fixierung des abtriebsseitigen Anschlussmittels 12, ist die radiale Lauffläche 16 durch eine Schulter 34 begrenzt. Ebenso kann ein Sicherungselement zur axialen Sicherung des abtriebsseitigen Anschlussmittels 12 eingesetzt werden.

Die Gelenkverbindung 10 weist zwischen dem Abtriebselement 17 und dem abtriebsseitigen Anschlussmittel 12 eine Schwenkbegrenzungseinrichtung 19 auf. Diese umfasst wenigstens ein Anschlagelement 23, 24 und das Antriebselement 22 und begrenzt einen freien Schwenkbereich α des abtriebsseitigen Anschlussmittels 12.

Gemäß der Figuren 1 und 3 ist die Schwenkbegrenzungseinrichtung 19 durch einen Schlitz 21, insbesondere radial verlaufenden Schlitz 21, gebildet, in welchen das Antriebselement 22 eingreift, das entlang des Schlitzes 21 verfahrbar ist. Das Antriebselement 22 ist durch den Motor 14 zumindest entlang des Schlitzes 21 schwenkbar angetrieben. Das Antriebselement 22 ist beispielsweise als stiftförmiges Antriebselement 22 ausgestaltet. Die den Schlitz 21 begrenzenden Stirnflächen, die zueinander beabstandet sind, bilden ein erstes Anschlagelement 23 und ein zweites Anschlagelement 24. Der freie Schwenkbereich α ist durch den Abstand zwischen den beiden Anschlagelementen 23, 24 bestimmt. Dadurch ist die freie Schwenkbewegung des abtriebsseitigen Anschlussmittels 12 in beide gegensinnigen Schwenkrichtungen begrenzt.

Durch Anliegen und Drehen des Antriebselementes 22 an einem der beiden Anschlagelemente 23, 24 der Schwenkbegrenzungseinrichtung 19 ist das abtriebsseitige Anschlussmittel 12 schwenkbar angetrieben. Dadurch ist ein angetriebener Schwenkbereich δ gebildet.

Der angetriebene Schwenkbereich δ des abtriebsseitigen Anschlussmittels 12 ist durch die Endanschläge 26, 27 begrenzt. Diese sind an dem antriebsseitigen Anschlussmittel 11 oder dem Gehäuse 13 vorgesehen und begrenzen einen einstellbaren Schwenkbereich des abtriebsseitigen Anschlusselementes 12. Der einstellbare Schwenkbereich α entspricht dem angetriebenen Schwenkbereich δ. An dem antriebsseitigen Anschlussmittel 11 oder dem Gehäuse 13 sind mehrere Bohrungen 28 (Figur 1) ringförmig und gleichmäßig zueinander beabstandet vorgesehen, in welche die Endanschläge 26, 27 in unterschiedlichen Positionen einsetz- oder einschraubbar sind. Alternativ können die Endanschläge 26, 27 auch entfallen, sodass eine Drehung des abtriebsseitigen Anschlussmittels 12 um 360 Grad ermöglicht ist oder der einstellbare Schwenkbereich durch die Motorsteuerung bestimmt wird.

An der Gelenkverbindung 10 ist ein Sensor 25 vorgesehen, welcher beispielsweise als Lagesensor ausgebildet ist. Durch diesen Sensor 25 ist eine Winkelstellung des abtriebsseitigen Anschlussmittels 12 und/oder eine Position des Antriebselementes 22 erfassbar. Der Sensor 25 ist mit einer nicht näher dargestellten elektronischen Regelung gekoppelt, welche den Motor 14 ansteuert. Bei der angetriebenen Schwenkbewegung des abtriebsseitigen Anschlussmittels 12 kann beispielsweise durch den Sensor 25 eine Winkelstellung des abtriebsseitigen Anschlussmittels 12 erkannt werden, sodass bei Erreichen einer bestimmten Winkelstellung oder einer Endposition 41, 42 des abtriebsseitigen Anschlussmittels 12, der Motor 14 abgeschaltet und die angetriebene Schwenkbewegung beendet wird.

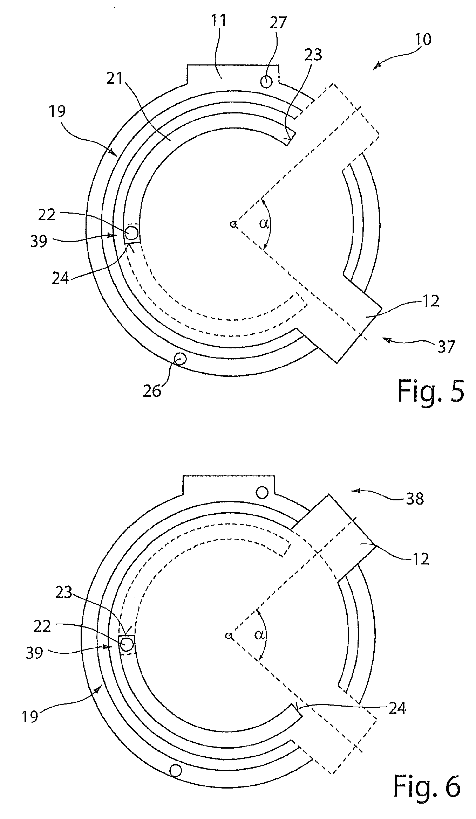

Figur 4 zeigt eine schematische Draufsicht der Gelenkverbindung 10 in einer Ausgangslage 36 des abtriebsseitigen Anschlussmittels 12 und einer Ausgangsposition 39 des Antriebselementes 22. Diese Ausgangslage 36 des abtriebsseitigen Anschlussmittels 12 und die Ausgangsposition 39 des Antriebselementes 22 kann sowohl für das Antriebselement 17 als auch für das abtriebsseitige Anschlussmittel 12 eine Mittelstellung darstellen. Aus dieser Mittelstellung ist das abtriebsseitige Anschlussmittel 12 in zwei gegensinnige Schwenkrichtungen frei schwenkbar, wobei der freie Schwenkbereich α durch die Schwenkbegrenzungseinrichtung 19 begrenzt ist. Die Lage des freien Schwenkbereiches α des abtriebsseitigen Anschlussmittels 12 ist durch die Position des Antriebselementes 22 bestimmt. Durch Drehung des Abtriebselementes 17 ändert sich die Position des Antriebselementes 22 in radialer Richtung um die Drehachse 18 relativ zum antriebsseitigen Anschlussmittel 11, sodass die Lage des freien Schwenkbereiches α innerhalb des durch die Endanschläge 26, 27 begrenzten einstellbaren Schwenkbereiches verändert wird. Die Länge des Schlitzes 21 bestimmt den verbleibenden freien Schwenkbereiche α und stellt in Figur 5 eine exemplarische Länge dar, welche an den jeweiligen Anwendungsfall individuell anpassbar ist.

Ausgehend von der Ausgangslage 36 des abtriebsseitigen Anschlussmittels 12 in Figur 4, ist dieses in zwei gegensinnige Schwenkrichtungen jeweils um einen Schwenkwinkel von 0,5 x α in eine Endlage 37 gemäß Figur 5 und eine Endlage 38 gemäß Figur 6 des freien Schwenkbereiches α schwenkbar. Die beiden Endlagen 37, 38 des freien Schwenkbereiches α sind dadurch festgelegt, dass das Antriebselement 22 mit einem der beiden Anschlagelemente 26, 27 der Schwenkbegrenzungseinrichtung 19 in Anlage gebracht ist.

Figur 5 zeigt in einer schematischen Draufsicht der Gelenkverbindung 10 die erste Endlage 37 des abtriebsseitigen Anschlussmittels 12 innerhalb des freien Schwenkbereiches α. In dieser ersten Endlage 37 ist das abtriebsseitige Anschlussmittel 12 um den freien Schwenkwinkel 0,5 x α aus der Ausgangslage 37 gemäß Figur 4, in eine erste Schwenkrichtung geschwenkt. Das Antriebselement 22 ist dabei weiterhin feststehend in der Ausgangsposition 39 angeordnet. Die Endlage 37 ist dadurch definiert, dass das zweite Anschlagelement 24 der Schwenkbegrenzungseinrichtung 19 mit dem Antriebselement 22 in Anlage gebracht ist.

Die Lage des freien Schwenkbereiches α und somit auch die erste Endlage 37 des abtriebsseitigen Anschlussmittels 12 ist durch die Position des Antriebselementes 22 bestimmt, welche durch Drehung des Abtriebselementes 17 mittels Motor 14 veränderbar ist. Dadurch ist die Lage des freien Schwenkbereiches α und somit auch die Position der ersten Endlage 37 des abtriebsseitigen Anschlussmittels 12, in den Grenzen des einstellbaren Schwenkbereiches δ frei veränderbar. Der einstellbare Schwenkbereich δ ist dabei durch die Anordnung der Endanschläge 26, 27 oder die elektronische Regelung bestimmt.

In Figur 6 ist in einer weiteren schematischen Draufsicht der Gelenkverbindung 10 abweichend zu Figur 5, das abtriebsseitige Anschlussmittel 12 in einer zweiten Endlage 38 innerhalb des freien Schwenkbereiches α dargestellt. In dieser zweiten Endlage 38 ist das abtriebsseitige Anschlussmittel 12 um den Schwenkwinkel 0,5 x α aus der Ausgangslage 36 gemäß Figur 4, in eine zweite Schwenkrichtung ausgelenkt, welche gegensinnig zur ersten Schwenkrichtung gemäß Figur 5 gerichtet ist. In der zweiten Endlage 38 ist das Antriebselement 22 mit dem ersten Anschlagelement 23 der Schwenkbegrenzungseinrichtung 19 in Anlage gebracht. Das Antriebselement 22 ist dabei weiterhin feststehend in der Ausgangsposition 39 angeordnet. Auch für die Darstellung in Figur 6 gilt, dass die Lage des freien Schwenkbereiches α und somit auch die Position der zweiten Endlage 38 des abtriebsseitigen Anschlussmittels 12, innerhalb des einstellbaren Schwenkbereiches durch Drehung des Abtriebselementes 17 um die Drehachse 18 frei veränderbar ist.

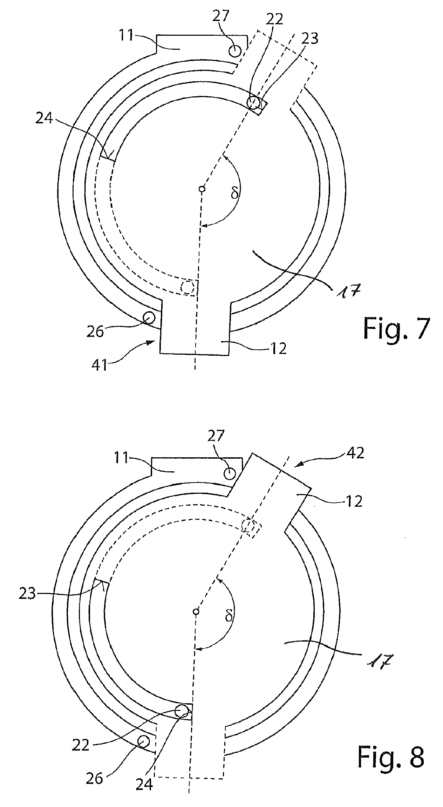

Figur 7 zeigt eine schematische Draufsicht der Gelenkverbindung 10 in einer ersten Endposition 41 des abtriebsseitigen Anschlussmittels 12 im angetriebenen Schwenkbereich δ. Der angetriebene Schwenkbereich δ und der freie Schwenkbereich α sind zumindest abschnittsweise überlagert, sodass die Schwenkbewegung des abtriebsseitigen Anschlussmittels 12 innerhalb des freien Schwenkbereiches α auch durch eine angetriebene Schwenkbewegung ausgeführt werden kann.

In der ersten Endposition 41 ist das abtriebsseitige Anschlusselement 12 in Anlage an den ersten Endanschlag 26 gebracht. Ausgehend von der Ausgangsposition 39 des Antriebselementes 22 und der Ausgangslage 36 des abtriebsseitigen Anschlussmittels 12 gemäß Figur 4, wird die erste Endposition 41 des abtriebsseitigen Anschlussmittels 12 erreicht, indem das Antriebselement 22 durch Drehung des Abtriebselementes 17 zunächst in Anlage an das erste Anschlagelement 23 der Schwenkbegrenzungseinrichtung 19 gebracht wird. Durch anschließendes weiteres Drehen des Antriebselementes 22 (im Uhrzeigersinn) wird eine angetriebene Schwenkbewegung des abtriebsseitigen Anschlussmittels 12 ausgeführt, bis das abtriebsseitige Anschlussmittel 12 die erste Endposition 41 erreicht und die elektronische Regelung den Motor 14 stillsetzt. Die Anordnung des ersten Endanschlages 26 oder die elektronische Regelung bestimmt die Lage der ersten Endposition 41. Eine unmittelbare Berührung zwischen dem abtriebsseitigen Anschlussmittel 12 und dem ersten Endanschlag 26 wird bei der angetriebenen Schwenkbewegung vermieden, indem die elektronische Regelung die angetriebene Schwenkbewegung des abtriebsseitigen Anschlussmittels 12 kurz vor Erreichen des ersten Endanschlages 26 zum Stillstand bringt, indem der Motor 14 stillgesetzt wird. Der erste Endanschlag 26 kann als zusätzliches mechanisches Sicherheitselement dienen, falls ein rechtzeitiger Stillstand des abtriebsseitigen Anschlussmittels 12 vor Erreichen des ersten Endanschlages 26 durch die elektronische Regelung nicht erfolgen sollte.

Alternativ wird die erste Endposition 41 des abtriebsseitigen Anschlussmittels 12 (Figur 7) erreicht, indem, ausgehend von der Ausgangsposition 39 des Antriebselementes 22 und der Ausgangslage 36 des abtriebsseitigen Anschlussmittels 12 gemäß Figur 4, das abtriebsseitige Anschlussmittel 12 zunächst eine freie Schwenkbewegung (im Uhrzeigersinn) ausführt, bis der erste Endanschlag 23 der Schwenkbegrenzungseinrichtung 19 in Anlage mit dem Antriebselement 22 gebracht ist. Das abtriebsseitige Anschlussmittel 12 befindet sich dadurch in der ersten Endlage 37 gemäß Figur 5. Bei Erreichen der Endlage 37 wird durch den Sensor ein Signal ausgegeben, welches über die elektronische Regelung den Motor 14 ansteuert. Durch den Motor 14 wird die Drehbewegung des Abtriebselementes 17 angetrieben, sodass über das Antriebselement 22 das abtriebsseitige Anschlussmittel 12 die angetriebene Schwenkbewegung ausführt, bis dieses die Endposition 41 gemäß Figur 7 erreicht und der Motor 14 durch die elektronische Regelung ausgeschaltet wird.

Während der angetriebenen Schwenkbewegung kann das Antriebselement 22 kontinuierlich die Position ändern, sodass die Lage des freien Schwenkbereiches α gleichzeitig kontinuierlich in die jeweilige Schwenkrichtung des Antriebselements 22 verschoben wird. Während der angetriebenen Schwenkbewegung ist eine freie Schwenkbewegung des abtriebsseitigen Anschlussmittels 12 innerhalb des freien Schwenkbereiches α weiterhin ermöglicht.

Ist das abtriebsseitige Anschlussmittel 12 durch die angetriebene Schwenkbewegung in Anlage zu einem der Endanschläge 26, 27 gebracht, ist die freie Schwenkbewegung des abtriebsseitigen Anschlussmittels 12 durch den Endanschlag 26, 27 und der Position des Antriebselements 22 verhindert.

Figur 8 zeigt eine weitere schematische Draufsicht der Gelenkverbindung 10. Diese Darstellung zeigt das abtriebsseitige Anschlussmittel 12 in einer zweiten Endposition 42 des angetriebenen Schwenkbereiches δ.

Ausgehend von der ersten Endposition 41 gemäß Figur 7, wird die zweite Endposition 42 gemäß Figur 8 dadurch erreicht, dass das Antriebselement 22 aus der Position gemäß Figur 7, durch Drehung des Abtriebselementes 17 (gegen den Uhrzeigersinn) zunächst in Anlage mit dem zweiten Anschlagelement 24 der Schwenkbegrenzungseinrichtung 19 gebracht wird. Anschließend erfolgt durch weiteres Drehen des Abtriebselementes 17 eine durch das Antriebselement 22 angetriebene Schwenkbewegung des abtriebsseitigen Anschlussmittels 12 (gegen den Uhrzeigersinn), bis dieses die zweite Endposition 42 erreicht. Die zweite Endposition 42 des angetriebenen Schwenkbereiches wird durch die Lage des zweiten Endanschlages 27 oder ebenfalls durch die elektronische Regelung bestimmt. Eine Berührung zwischen dem abtriebsseitigen Anschlussmittel 12 und dem zweiten Endanschlag 27 wird auch bei Erreichen der zweiten Endposition 42 durch die elektronische Regelung vermieden.

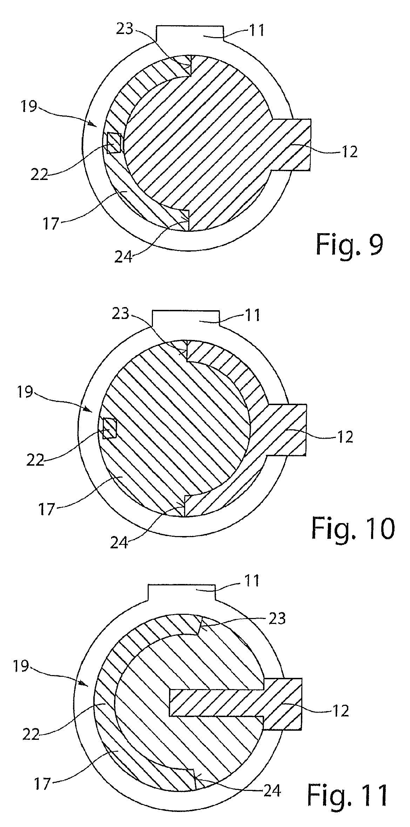

Figur 9 zeigt eine schematische Draufsicht einer alternativen Ausführungsform einer Schwenkbegrenzungseinrichtung 19 der Gelenkverbindung 10. Bei dieser Ausführungsform ist das abtriebsseitige Anschlussmittel 12 scheibenförmig ausgestaltet und weist an zwei sich gegenüberliegenden Abschnitten jeweils ein Absatz auf, durch welchen die beiden Anschlagelemente 23, 24 gebildet sind.

Figur 10 zeigt eine weitere schematische Draufsicht einer zweiten alternativen Ausführungsform der Schwenkbegrenzungseinrichtung 19 der Gelenkverbindung 10, bei welcher das abtriebsseitige Anschlussmittel 12 halbkreisförmig ausgebildet ist und schwenkbar um die Drehachse 18 herum angeordnet ist. Durch das halbkreisförmige, abtriebsseitige Anschlussmittel 12 sind zwei Endabschnitte gebildet, welche die Anschlagelemente 23, 24 darstellen und an welche das Antriebselement 22 bei einer freien Schwenkbewegung des abtriebsseitigen Anschlussmittels 12 oder einer Drehbewegung des Antriebselementes 22 in Anlage gebracht wird.

Aus Figur 11 geht eine schematische Draufsicht einer dritten alternativen Ausführungsform der Schwenkbegrenzungseinrichtung 19 der Gelenkverbindung 10 hervor. Bei dieser Ausführungsform bildet das abtriebsseitige Anschlussmittel 12 einen Arm, der schwenkbar an der Drehachse 18 angeordnet ist. Das Antriebselement 22 ist im Wesentlichen halbkreisförmig ausgestaltet, wobei die beiden Endabschnitte des halbkreisförmigen Antriebselementes 22 die Anschlagelemente 23, 24 bilden, welche bei einer Drehbewegung des Antriebselementes 22 oder bei einer Schwenkbewegung des abtriebsseitigen Anschlussmittels 12 in Anlage mit dem armförmigen Anschlussmittel 12 gebracht werden.

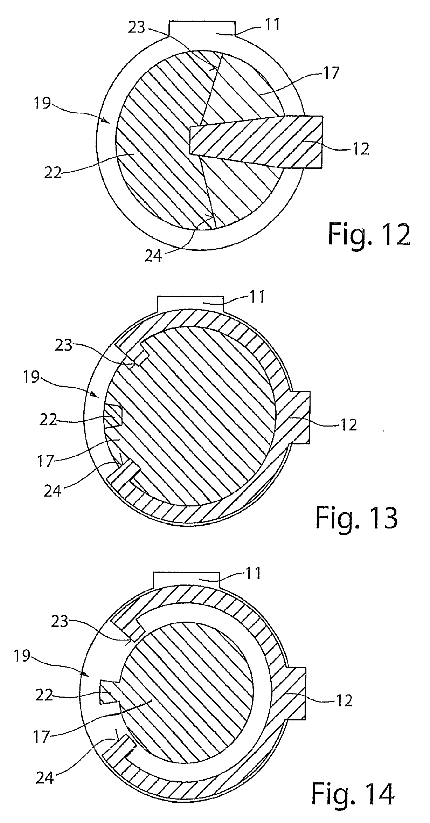

Aus der schematischen Draufsicht einer vierten alternativen Ausführungsform der Schwenkbegrenzungseinrichtung 19 der Gelenkverbindung 10 gemäß Figur 12, geht ein kreissegmentförmiges Antriebselement 22 hervor, welches mit den durch das kreissegmentförmige Antriebselement 22 gebildeten Anschlagelementen 23, 24 bei einer Drehbewegung in Anlage mit einem armförmigen, abtriebsseitigen Anschlussmittel 12 gebracht wird um eine angetriebene Schwenkbewegung auszuführen oder eine freie Schwenkbewegung des abtriebsseitigen Anschlussmittels 12 zu begrenzen.

Figur 13 zeigt eine fünfte alternative Ausführungsform der Schwenkbegrenzungseinrichtung 19 der Gelenkverbindung 10 in einer schematischen Draufsicht. Bei dieser Ausführungsform bildet das abtriebsseitige Anschlussmittel 12 im Wesentlichen einen Dreiviertelkreis, welches an den jeweiligen Endabschnitten des Dreiviertelkreises des Anschlussmittels 12 jeweils ein Anschlagelement 23, 24 aufweist. Dieses ist jeweils durch einen nach innen weisenden Absatz ausgestaltet. Bei einer Schwenkbewegung wird das Antriebselement 22 mit einem der Anschlagelemente 23, 24 in Anlage gebracht.

Aus Figur 14 geht eine schematische Draufsicht einer sechsten alternativen Ausführungsform der Schwenkbegrenzungseinrichtung 19 der Gelenkverbindung 10 hervor. Bei dieser Ausführungsform ist das abtriebsseitige Anschlussmittel 12 ebenso wie das abtriebsseitige Anschlussmittel 12 in Figur 13 ausgebildet. Das Antriebselement 22 ist dagegen scheibenförmig ausgestaltet und um die Drehachse 18 drehbar gelagert angeordnet. Dieses weist einen zahnförmigen Mitnehmer auf, welcher das Antriebselement 22 bildet und bei einer Drehbewegung des Antriebselementes 22 in Anlage zu einem der Anschlagelemente 23, 24 des abtriebsseitigen Anschlussmittels 12 gebracht wird.

Die vorstehend beschriebene Gelenkverbindung kann universell eingesetzt werden, sodass sowohl eine Verwendung als Orthese oder zur gezielten und kontrollierten Rehabilitation von Gelenken möglich ist als auch ein Einsatz als Arbeitsgerät zum kraftunterstütztem Anheben oder Absenken von Lasten.

## Patentansprüche

1. Gelenkverbindung (10) mit einer Antriebseinrichtung (15), insbesondere für die Bewegungsunterstützung von biomechanischen Gelenken für eine Orthese, mit einem antriebsseitigen Anschlussmittel (11) und einem abtriebsseitigen Anschlussmittel (12), die schwenkbar zueinander gelagert sind, wobei die Antriebseinrichtung (15) einen Motor (14) aufweist, zu dessen Gehäuse (13) das antriebsseitige Anschlussmittel (11) feststehend angeordnet ist und das abtriebsseitige Anschlussmittel (12) an dessen Abtriebselement (17) oder zum Gehäuse (13) drehbar angeordnet ist,
**dadurch gekennzeichnet,**
**dass** eine Schwenkbegrenzungseinrichtung (19) zwischen dem Abtriebselement (17) und dem abtriebsseitigen Anschlussmittel (12) wirkt, welche wenigstens ein Anschlagelement (23, 24) und wenigstens ein an dem zumindest einen Anschlagelement (23, 24) zur Anlage kommendes Antriebselement (22) umfasst, und welche einen freien Schwenkbereich (α) des abtriebsseitigen Anschlussmittels (12) zum Abtriebselement (17) begrenzt, dass das Abtriebselement (17) mittels der Schwenkbegrenzungseinrichtung (19) das abtriebsseitige Anschlussmittel (12) drehbar innerhalb eines angetriebenen Schwenkbereiches (δ) antreibt und
**dass** der freie Schwenkbereich (α) und der angetriebene Schwenkbereich (δ) zumindest teilweise überlagert sind.

2. Gelenkverbindung nach Anspruch 1, **dadurch gekennzeichnet, dass** das abtriebsseitige Anschlussmittel (12) von dem Abtriebselement (17) durch eine Radiallagerung (33) drehbar aufgenommen ist.

3. Gelenkverbindung nach Anspruch 1, **dadurch gekennzeichnet, dass** das abtriebsseitige Anschlussmittel (12) von dem Gehäuse (13) oder dem antriebsseitigen Anschlussmittel (11) durch eine Radiallagerung (33) drehbar aufgenommen ist.

4. Gelenkverbindung nach Anspruch 2, **dadurch gekennzeichnet, dass** das abtriebsseitige Anschlussmittel (12) unmittelbar auf einer radialen Lauffläche (16) des Abtriebselementes (17), des Gehäuses (13) oder dem antriebsseitigen Anschlussmittel (11) abgestützt ist.

5. Gelenkverbindung nach Anspruch 4, **dadurch gekennzeichnet, dass** das abtriebsseitige Anschlussmittel (12) durch eine Schulter (34) oder ein Sicherungselement zur Lauffläche (16) des Abtriebselementes (17), des Gehäuses (13) oder dem antriebsseitigen Anschlussmittel (11) in axialer Richtung gesichert ist.

6. Gelenkverbindung nach Anspruch 1, **dadurch gekennzeichnet, dass** das antriebsseitige Anschlussmittel (11) und das abtriebsseitige Anschlussmittel (12) um eine gemeinsame Achse schwenkbar gelagert sind und vorzugsweise das abtriebsseitige Anschlussmittel (12) und das Abtriebselement (17) um eine gemeinsame Drehachse (18) schwenkbar gelagert sind, welche insbesondere der gemeinsamen Achse des antriebsseitigen Anschlussmittels (11) und des abtriebsseitigen Anschlussmittels (12) entspricht.

7. Gelenkverbindung nach Anspruch 1, **dadurch gekennzeichnet, dass** die Schwenkbegrenzungseinrichtung (19) zwei zueinander beabstandete Anschlagelemente (23, 24), und wenigstens ein an dem zumindest einem Anschlagelement (23, 24) zur Anlage kommendes Antriebselement (22) umfasst.

8. Gelenkverbindung nach Anspruch 7, **dadurch gekennzeichnet, dass** das Antriebselement (22) an dem Abtriebselement (17) angeordnet ist und die wenigstens zwei Anschlagelemente (23, 24) an dem abtriebsseitigen Anschlussmittel (12) vorgesehen sind oder
das Antriebselement (22) an dem abtriebsseitigen Anschlussmittel (12) angeordnet ist und die wenigstens zwei Anschlagelemente (23, 24) an dem Abtriebselement (17) vorgesehen sind.

9. Gelenkverbindung nach Anspruch 7, **dadurch gekennzeichnet, dass** die Anschlagelemente (23, 24)
durch einen Schlitz (21) oder eine Nut begrenzende Stirnflächen gebildet sind, welche an dem abtriebsseitigen Anschlussmittel (12) vorgesehen ist und in welche das vorzugsweise stiftförmige Antriebselement (22) eingreift oder durch an dem abtriebsseitigen Anschlussmittel (12) vorgesehene Zähne, Laschen oder Anschläge gebildet sind oderdurch ein abtriebsseitiges kreissegmentförmiges Anschlussmittel (12) gebildet sind.

10. Gelenkverbindung nach Anspruch 7, **dadurch gekennzeichnet, dass** beim Anliegen des Antriebselementes (22) an dem zumindest einem Anschlagelement (23, 24) das abtriebsseitige Anschlussmittel (12) in eine der beiden Drehrichtungen des Abtriebselementes (17) innerhalb eines angetriebenen Schwenkbereiches (δ) schwenkbar angetrieben ist.

11. Gelenkverbindung nach Anspruch 1, **dadurch gekennzeichnet, dass** die Antriebseinrichtung (15) zwischen einem Rotor (32) des Motors (14) und dem damit wirkverbundenen Abtriebselement (17) ein Getriebe (31) aufweist, welches insbesondere als Untersetzungsgetriebe ausgebildet ist.

12. Gelenkverbindung nach Anspruch 1, **dadurch gekennzeichnet, dass** als Motor (14) ein schnelllaufender Elektromotor vorgesehen ist.

13. Gelenkverbindung nach Anspruch 1, **dadurch gekennzeichnet, dass** wenigstens ein Sensorelement (25) zur Detektion der Stellposition des wenigstens einen Antriebselementes (22) und/oder der Winkelstellung des abtriebsseitigen Anschlussmittels (12) und zur Ansteuerung des Motors (14) vorgesehen ist.

14. Gelenkverbindung nach Anspruch 1, **dadurch gekennzeichnet, dass** wenigstens ein Sensor, vorzugsweise ein Drehmomentsensor, Dehnungsmessstreifen, Elektromyographie-Sensor (EMG-Sensor) oder eine externe Druckplatine, zur Ansteuerung des Motors (14) vorgesehen ist.

15. Gelenkverbindung nach Anspruch 1, **dadurch gekennzeichnet, dass** der freie Schwenkbereich (α) des abtriebsseitigen Anschlussmittels (12) gleich groß oder kleiner ist als ein einstellbarer Schwenkwinkel (δ) des abtriebsseitigen Anschlussmittels (12).

16. Gelenkverbindung nach Anspruch 15, **dadurch gekennzeichnet, dass** der einstellbare Schwenkwinkel des abtriebsseitigen Anschlusselementes (12) durch
wenigstens ein, vorzugsweise zwei Endanschläge (26, 27) am Gehäuse (13) oder am antriebsseitigen Anschlussmittel (11) oder
eine elektronische Regelung des Motors (14) begrenzt ist.

17. Verfahren zur Ansteuerung einer Schwenkbewegung von einem abtriebsseitigen Anschlussmittel (12) relativ zu einem antriebsseitigen Anschlussmittel (11) einer Gelenkverbindung (10) mit einer Antriebseinrichtung (15) nach einem der Ansprüche 1 bis 15, **dadurch gekennzeichnet,**
- **dass** ein Motor (14) der Antriebseinrichtung (15) durch Signale eines Sensors angesteuert wird,
- **dass** der Motor (14) eine Drehbewegung des Antriebselementes (22) ansteuert, sodass dieses zur Anlage an einem Anschlagelement (23, 24) einer Schwenkbegrenzungseinrichtung (19) übergeführt wird, welche zwischen dem Abtriebselement (17) und dem abtriebsseitigen Anschlussmittel (12) wirkt und das abtriebsseitige Anschlussmittel (12) geschwenkt wird oder bei Anlage des Antriebselementes (22) am Anschlagelement (23, 24) das abtriebsseitige Anschlussmittel (12) geschwenkt wird und
- **dass** der Motor (14) zum Antrieb der Schwenkbewegung des abtriebsseitigen Anschlussmittels (12) bei Erreichen einer Endposition (41, 42) innerhalb eines angetriebenen Schwenkbereiches des Antriebselementes (22) oder bei Anlage an einem dem angetriebenen Schwenkbereich zugeordneten Endanschlag (26, 27) stillgesetzt wird.

18. Verfahren nach Anspruch 17, **dadurch gekennzeichnet, dass** als Sensor zur Ansteuerung des Motors (14) ein Drehmomentsensor, Dehnungsmessstreifen, Elektromyographiesensor (EMG-Sensor) oder eine externe Druckplatine eingesetzt und der Sensor durch ein Belastung, einen muskulären oder nervalen Impuls oder eine aktive Betätigung der Druckplatine aktiviert wird.

19. Verfahren nach Anspruch 17, **dadurch gekennzeichnet, dass** nach einer kraftunterstützten Schwenkbewegung des abtriebsseitigen Anschlussmittels (12) innerhalb des angetriebenen Schwenkbereiches, der Motor (14) angesteuert wird, sodass das Antriebselement (22) in eine Ausgangsposition (39) zurückgeführt wird, welche vorzugsweise mittig zwischen zwei Endposition (41, 42) des angetriebenen Schwenkbereichs oder zwischen den Endanschlägen (26, 27) liegt.

20. Verfahren nach Anspruch 17, **dadurch gekennzeichnet, dass** die Lage des freien Schwenkbereiches des abtriebsseitigen Anschlussmittels (12) durch die Ausgangsposition (39) oder einer davon abweichenden Stellposition des Antriebselementes (22) festgelegt ist.
